# EUROPEAN PATENT APPLICATION

(11) **EP 0 554 653 A2**
(43) Date of publication of application: **11.08.1993**
(21) Application number: 93100012.9
(22) Date of filing: 04.01.1993
(51) Int. Cl.: A61L 17/00

(54) **Multicolored surgical braid**

(30) Priority: 24.01.1992 US 825245
(71) Applicant: AMERICAN CYANAMID COMPANY, Wayne, NJ 07470-8426 (US)
(72) Inventor: McCusker, Edward J., Danbury, Connecticut 06813 (US)
(74) Representative: Wächtershäuser, Günter, Prof. Dr.

(57) **Abstract**

A multicolored braided surgical suture is woven in one embodiment in a spiral multi-color braid. The suture is braided by moving thread carriers from position to position around a circular path, with each carrier moving from a present position to a succeeding position which is at least two positions removed from its present position. Such spiral braided sutures are advantageously produced without core filaments, providing benefits in strength, smoothness, pliability, cylindrical uniformity without the discontinuity of properties characteristic of conventionally braided cored sutures and visibility. The surgical suture is woven in a multi-color lattice braid in another embodiment, providing a plurality of distributed core passageways for individual core fibers.

## Description

The present invention relates to surgical sutures and, more particularly, to braided surgical sutures which obviate the need for a central fiber core and are composed of at least two colors of fiber to increase their visibility.

Multi-filament sutures consisting of a plurality of braided filaments of a fine gauge are well known in the art. These braided sutures provide advantages, such as increased pliability, over monofilament surgical sutures. However, since braided sutures often lack substantial tensile strength, the braided filaments are conventionally braided in a tubular sheath around a core of longitudinally extended threads.

Braided sutures with central core threads have, nevertheless, been found to exhibit certain disadvantages. One disadvantage is that the tensile strength of the suture is not evenly distributed between the braided sheath and the central core threads. As a result, the sheath and the core will respond differently to the application of tensile forces when the sutures are stretched. The braided sheath will respond to forces independently of the central core threads, causing the central core threads to move longitudinally relative to the surrounding sheath. The core threads also tend to flatten and redistribute themselves within the sheath instead of maintaining the desired rounded cross-sectional shape of the suture. It would be preferable to distribute more uniformly the tensile forces throughout the suture so that all of the fibers of the suture will respond in unison to the tensile forces without distorting the normal shape of the suture.

Conventional braided sutures also formed interstices where the braided fibers overlap and cross each other. These interstices can trap and retained moisture, which deteriorates sutures made of certain materials and can also lead to infection within the braided suture. Accordingly, braided sutures are preferred that do not have interstices or passageways which can trap and retained moisture prior to use.

Braided sutures are conventionally made of a single color. Despite differing characteristics between surgical sutures, such as the material used for the suture, the diameter or non-cylindrical shape of the suture and even the type of braid of the suture, sutures of such different characteristics that have a single color can be almost indistinguishable from each other, unless a close visual inspection is made. It would be preferable to provide a surgical braid comprised of at least two colors of fiber to make each suture more highly visible during surgery and distinguishable from other surgical sutures.

A braided suture in which the filaments or threads are braided in a spiral pattern without a central fiber core or in a lattice pattern is shown in the prior art. However, the filaments or threads are composed of a single color, and thus not readily distinguishable from one another.

It is a principle object of the present invention to improve upon and enhance known surgical sutures.

It is an object of the present invention to provide a braided surgical suture.

It is a further object of the invention to provide a braided surgical suture that is formed of at least two colors of fibers.

It is still a further object of the invention to provide a braided surgical suture of different types of braids which can be distinguish by different multicolored patterns.

In accordance with the one aspect of the present invention, the braided surgical suture comprises a plurality of surgically compatible filaments woven in a spiral braid by moving filament dispensers to different positions around a closed loop, wherein an individual dispenser and a loop are moved from their current position to a succeeding position which is as least two positions removed from the current position, and wherein at least one filament dispenser has a different color fiber than the other filament dispensers.

In accordance with another aspect of the present invention, a braided surgical suture comprises a plurality of surgically compatible filaments woven in a lattice braid by moving filament dispensers in three closed loop paths, with first and second paths being generally oblong and crossing over each other at a central intersection, the third path passing through the ends of the first and second paths outside the central intersection, and wherein the filaments in at least one path are a different color than the filaments in the other paths.

These and other objects, aspects, features and advantages of the present invention will become apparent from the following detailed description of the preferred embodiments taken in conjunction with the accompanying drawings.

### DRAWINGS

Figure 1 is a side view of a conventionally braided suture;
Figure 2 is a perspective view of a conventionally braided suture;
Figure 3 is an side elevational view of a first variation of a multicolored spiral braided suture in accordance with a first embodiment of the present invention;
Figure 4 is an side elevational view of a second variation of a multicolored spiral braided suture in accordance with the first embodiment of the present invention;
Figure 5 illustrates the braiding pattern of a spiral braided suture in accordance with the first embodiment of the present invention;
Figure 6 illustrates a first variation of the braiding pattern of a multicolored lattice braided suture in accordance with a second embodiment of the present invention;
Figure 7 is an side elevational view of the lattice suture braided in the pattern shown in Figure 6, the view of all sides in this embodiment being the same;
Figure 8 illustrates a second variation of the braiding pattern of the multicolored lattice braided suture in accordance with the second embodiment of the present invention;
Figure 9 is a front side elevational view of the lattice suture braided in the pattern shown in Figure 8, the rear elevational view being identical thereto;
Figure 10 is a left side elevational view of the lattice suture braided in the pattern shown in Figure 8, the rear elevational view being identical thereto;
Figure 11 illustrates a third variation of the braiding pattern of the multicolored lattice braided suture in accordance with the second embodiment of the present invention;
Figure 12 is a front side elevational view of the lattice suture braided in the pattern shown in Figure 11, the rear elevational view being identical thereto; and
Figure 13 is a left side elevational view of the lattice suture braided in the pattern shown in Figure 11, the right side elevational view being identical thereto.

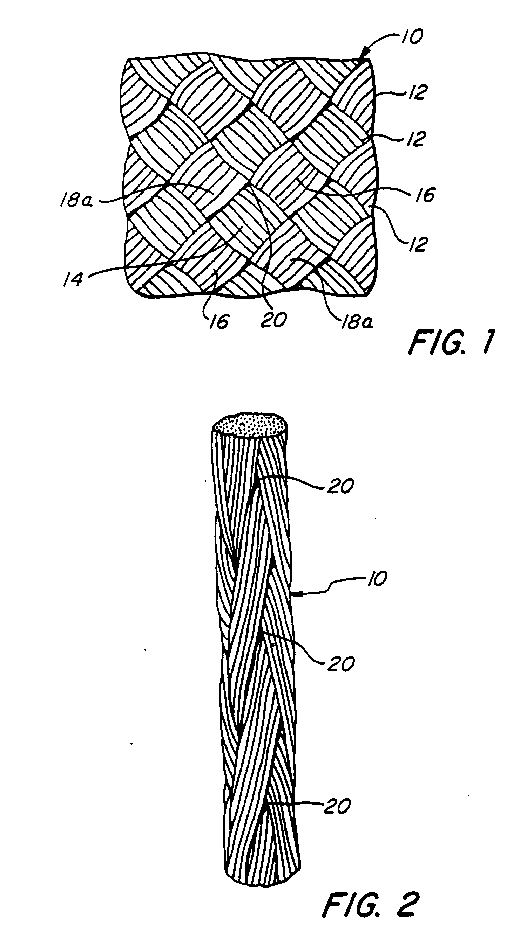

Figure 1 illustrates the outside of the braided sheath of conventional suture 10 showing the crossing pattern of braided threads 12. Each thread is composed of a number of individual fibers as indicated by the lines on each thread. Where each thread appears on the outside of the sheath it is seen to be orthogonally directed with respect to the thread it crosses over, the thread from beneath which it appears, and the thread it next crosses under. For instance, thread 14 is orthogonally directed with respect to thread 16 on either side of thread 14 where it crosses over thread 16. Thread 14 is also orthogonally directed with respect to thread 18a from beneath which it appears, and with respect to thread 18b which it next crosses under.

This orthogonal crossing relationship of the braided threads results in the formation of small interstices or voids 20 where the threads cross one another, as shown in Figures 1 and 2. These voids 20 entrap moisture which can lead to premature deterioration of the suture, and can also entrap bacteria and other sources of infection which could complicate healing of the wound.

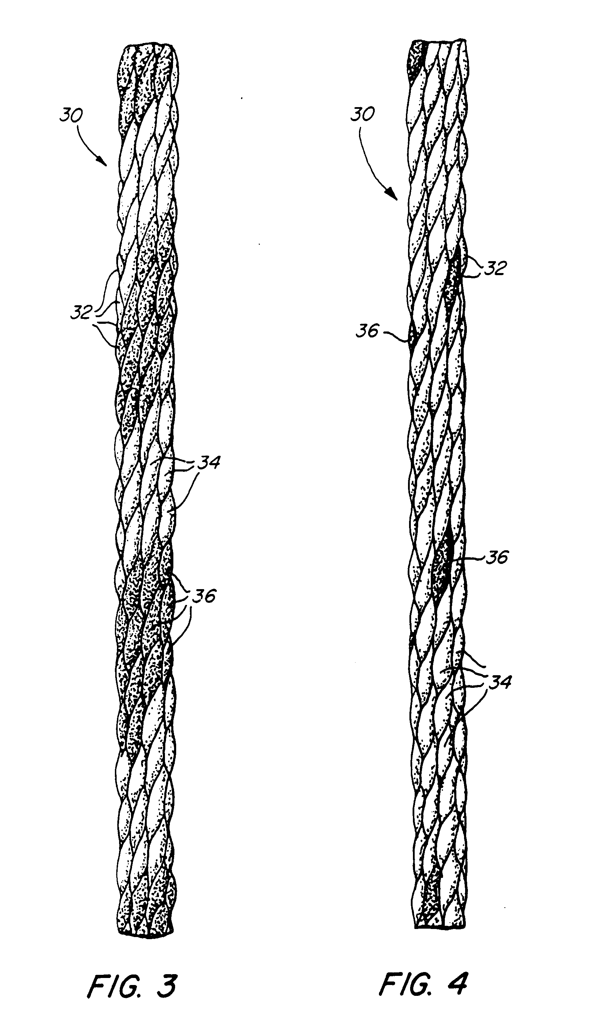

Figures 3 and 4 illustrate two variations of a spiral braided suture 30 in accordance with a first embodiment of the present invention. The multicolored sutures shown in Figures 3 and 4 are identical in structure and differ only in the way in which the multicolored pattern is incorporated into the suture. Accordingly, the following discussion applies eXally to the sutures shown in both Figures 3 and 4, except where noted. Threads 32 on the outside of the spiral braided suture are seen to be aligned in a spiral pattern which ascends from the lower left to the upper right in the drawings as the outer threads precess around the outer surface of the suture. As the pattern spirals, individual threads on the outer surface are in a parallel orientation with respect to each other and with respect to the longitudinal length of the suture as they continually reappear in the spiral pattern.

With all threads aligned in the parallel, offset spiral pattern shown in Figures 3 and 4, it may be seen that there are no voids or interstices formed on the outside surface of the suture. This is due to the parallel orientation of the threads, as opposed to the orthogonally directed crossing pattern of the threads of the conventionally braided suture of Figures 1 and 2. The parallel orientation of the outer appearing threads also provides a smoother feel to the suture, since the hand will sense the continuous, longitudinal orientation of the parallel threads as it is run along the suture. In addition, the interlocking of threads 32 causes the threads to move in unison as a contiguous structure, thereby uniformly distributing tensile forces when suture 30 is pulled or stressed.

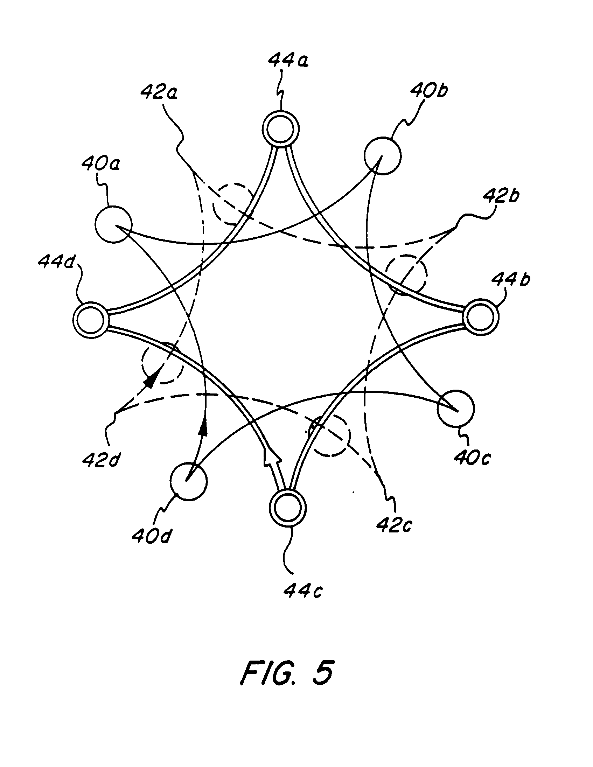

A spiral braided suture of the present invention is formed of four or more interwoven threads with at least one thread being a different color than the other three. Preferably, at least nine threads are braided in groups of three. A braiding pattern for a spiral braided suture of twelve threads, arranged in groups of four, is shown in Figure 5. In the illustrated pattern the carriers move sequentially in the same direction around the circular loop of carriers. As they move, each carrier moves from its present position to a succeeding position which is at least two positions removed from its present position. In Figure 5, each carrier moves to the third succeeding position around the loop. The twelve carriers are grouped into three groups of four carriers each. In the first group, carriers move in unison between positions 42a, 42b, 42c and 42d. The carrier at position 42a moves to position 42b, passing by positions 44a and 40b as it does so. As it moves, the carrier at position 42b is moving to position 42c bypassing positions 44b and 40c. At the same time the carrier at position 42c is moving to position 42d and the carrier at position 42d is moving to position 40a.

After these four carriers have moved to their new positions in unison, the carriers at positions 44a, 44b, 44c and 44d move to their succeeding positions. Then the carriers at positions 40b, 40c, 40d and 40a move to their succeeding positions. The sequence then repeats in the same fashion.

The multicolored braided suture shown in Figure 3 is achieved by making half of the total number of threads a different color than the other half of threads. Using the spiral braided suture woven by the braiding pattern shown in Figure 5 as an example, six of the twelve threads contained on the carriers would be of one color and the other six threads contained on the carriers would be of another color. The spiral braiding pattern shown in Figure 3 is formed of twenty threads, with ten threads of a first color 34 and ten threads of a second color 36 woven together to create a suture 30 with substantially equal amounts of the two colors in separate spiralling patterns.

On the other hand, the multicolored suture shown in Figure 4 is achieved by making one thread a different color than the other threads. The spiral braided pattern shown in Figure 4 is also formed of twenty threads, with nineteen threads being of a first color 34 and one thread being of a second color 36 woven together to create a suture 30 with a single thread of a different color spiralling therethrough.

The multicolored sutures shown in Figures 3 and 4 create a unique braid which makes sutures of one type easily distinguishable from other types of sutures.

An apparatus for executing the spiral braiding pattern illustrated in Figure 5 is disclosed in the prior art.

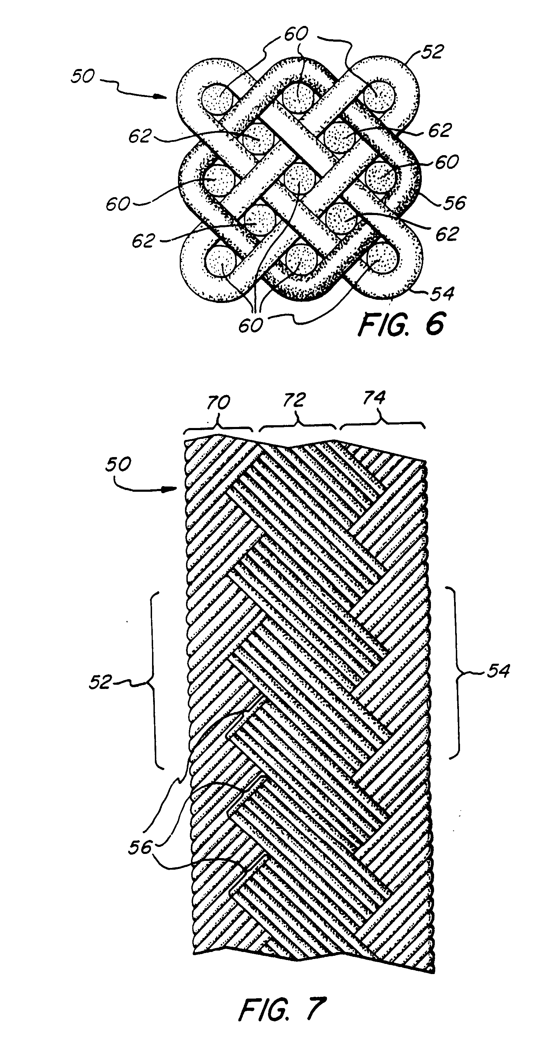

Figures 6 through 13 illustrate three variations of a second embodiment of the present invention directed to a multicolored lattice braided suture 50. The multicolored sutures shown in Figures 6 through 13 are identical in structure and differ only in the way in which the multicolored pattern is incorporated into the suture. Accordingly, the following discussion applies equally to the structure of the sutures shown in Figures 6 through 13, except where noted.

A cross-sectional view of lattice braided suture 50 of the present invention is shown in Figures 6, 8 and 11. Three or more threads are braided in a lattice pattern. One thread or group of threads traverses in path 52 a loop extending from the upper right to the lower left of the drawings. As the carrier or carriers dispensing thread on path 52 move around path 52, they alternately cross over and under the paths of the other threads that they encounter, the crossing pattern being determined by the times and locations of travel of the respective carriers. In a similar fashion a second carrier or carriers dispensing thread traverse a path 54 from the lower right to the upper left of the pattern. Like the first path, the thread dispensed from carriers travelling path 54 alternately crosses over and under the other paths it encounters. A third path 56 travels around the intersection of the first path and the second path. Like the first two paths, the thread dispensed from the carrier or carriers traversing path 56 alternately crosses over and under the threads of the other paths it encounters. In the present invention, at least one of the threads in the three paths is a different color than the others.

The lattice braid is seen to exhibit a generally square shape with rounded corners in cross-section. While the lattice braided suture has been found to provide less tensile strength than the spiral braided suture, the lattice braided suture can be strengthened by including individual core threads running longitudinally through the interlocking lattice. A number of core threads may be located at the positions indicated at 60 in the lattice, at the positions indicated at 62, or both. This uniform distribution of core threads throughout the lattice, which results in secure capture of the individual threads within the loops of the lattice, has been found to provide a uniform distribution of tensile forces throughout the suture.

The outside of the lattice braided suture 50 is shown in Figures 7, 9, 10, 12 and 13. The outer threads of the lattice are seen to be distributed in an angularly offset, generally parallel configuration. The drawings show the generally parallel alignment of threads 52, 54 and 56 on the outside of the suture, forming a substantially smooth, longitudinally extending outer thread surface on each side of the square configuration. The rounded corners shown on each side of the drawing are also seen to smoothly extend along the length of the suture.

The bi-colored lattice suture shown in Figures 6 and 7 is achieved by making the thread that travels along path 56 a different color than the threads that travel along oblong paths 52 and 54. This creates a lattice suture with a middle portion 72 having a different color than either side portion 70, 74 as shown in the omnidirectional side view of Figure 7.

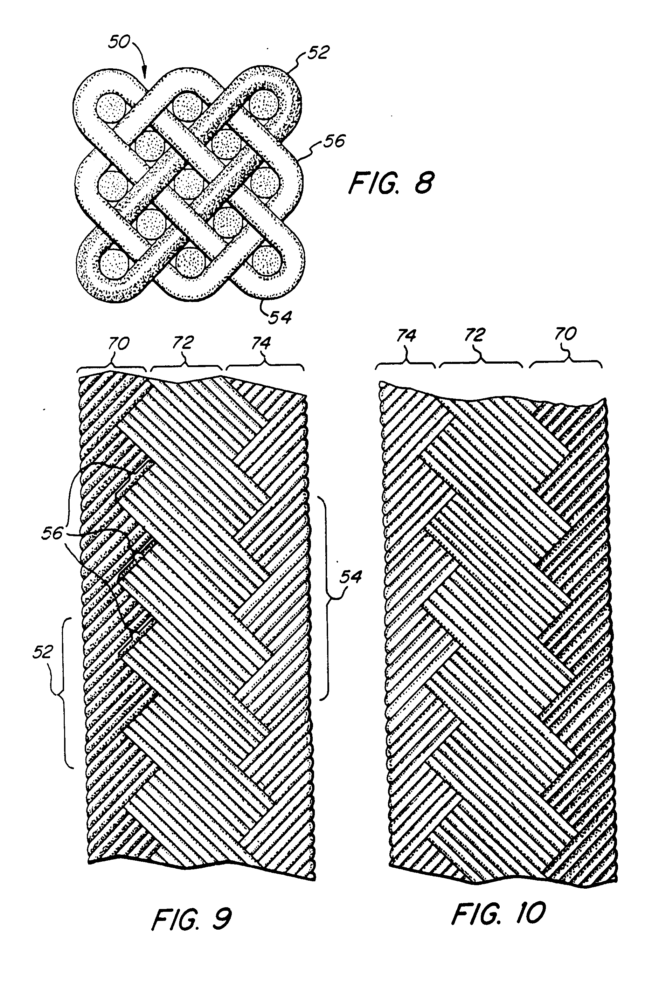

The bi-colored lattice suture shown in Figures 8 through 10 is achieved by making the thread that travels in path 52 a different color than the threads travelling in paths 54 and 56. This creates a lattice suture with diagonally opposed corners having a different color than the other portions of the lattice suture. Figures 9 and 10 show front and left side views, respectively, illustrating side portion 70 of a different color than the middle portion 72 and side portion 74.

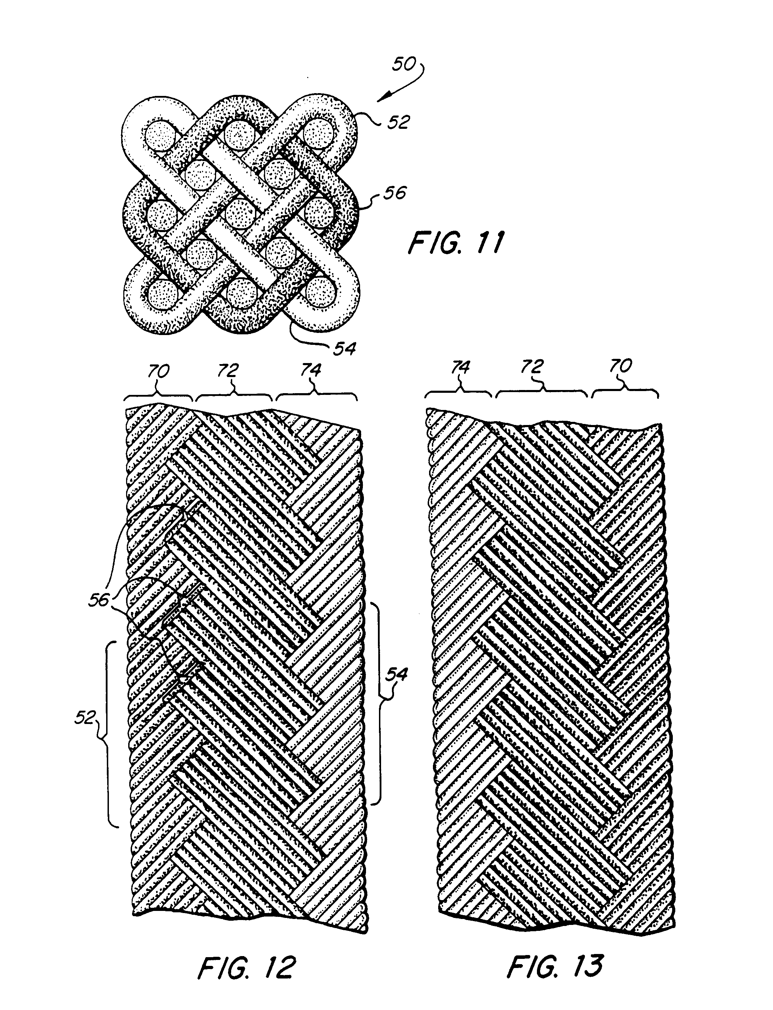

The tri-color lattice structure shown in Figures 11 through 13 is achieved by making the thread that travels in path 52 one color, the thread that travels in path 54 a second color and the thread that travels in path 56 a third color. This creates a lattice structure with each middle portion 72 and side portions 70, 74 having a different color from each other as shown in the side views of Figures 12 and 13.

The apparatus for executing the lattice braiding pattern as shown in Figures 6, 8 and 11 is disclosed in the prior art.

Spiral braided multi-color sutures of the present invention can be expected to provide a 20% improvement in smoothness over conventionally braided sutures, a 20% improvement in pliability, a 50% improvement in cylindrical uniformity, and a 700% improvement in visibility in the operative field. The improvement in smoothness is due to a parallel alignment of the suture threads on the outside of the spiral braided suture. The improvement in pliability is due to the thread crossovers of the spiral braid, which enhances fiber mobility and enables the individual threads in the spiral braided suture to easily move relative to each other as the suture is bent. And since there is no core to become misaligned or misshaped, cylindrical uniformity is improved. Improvements in breaking strength can also be expected for the spiral braided suture. The improvement in visibility is due to the use of different colored fibers. The unique visibility properties of this suture braid allow a surgeon to distinguish any one suture from other sutures. Different multicolor suture patterns can be selected by the surgeon for different portions of a surgical procedure depending on its complexity.

The lattice braided suture provides the capability of producing a high quality composite suture in which advantage is taken of the different characteristics of one type of material for the braid and another type of material for the core threads. The lattice braided suture is substantially more immune to the problem of core pop than the conventionally braided suture, since the core threads are distributed throughout the structure of the braid and are not positioned in a single central location. Both the spiral and lattice braided sutures have been found to exhibit less surface area exposed to ambient conditions, and hence less exposure to moisture than conventionally braided sutures.

Although specific embodiments of the present invention have been described above in detail, it will be understood that this description is merely for purposes of illustration. Various modifications of and equivalent structures corresponding to the disclosed aspects of the preferred embodiments in addition to those described above may be made by those skilled in the art without departing from the spirit of the present invention which is defined in the following claims, the scope of which is to be accorded the broadest interpretation so as to encompass such modifications and equivalent structures.

## Claims

1. A surgical suture comprising a plurality of compatible filaments braided into a spiral or lattice braid wherein at least one filament in said suture has a different colored fiber than the other filaments therein.

2. The surgical suture of claim 1 braided into a spiral braid by moving filament dispensers to different positions around a closed loop, wherein an individual dispenser in the loop is moved from its current position to a succeeding position which is at least two positions removed from said current position, and wherein at least one filament in said suture has a different colored fiber than the other filaments therein or into a lattice braid by moving filament dispensers in three closed loop paths, a first and second of said paths being generally oblong and crossing over each other at a central intersection, the third of said paths passing through the ends of said first and second paths outside said central intersection, wherein the filaments in at least one path are a different color than the filaments in the other paths.

3. The surgical suture of claim 2 braided into a spiral braid, wherein the portions of said filaments which are visible on the outside of the said braided suture are oriented substantially parallel to each other and are distributed in patterns which spiral around the outside of said suture, and optionally wherein said surgically compatible filaments are without any central, longitudinally extending core filaments.

4. The surgical suture of claim 2 or 3 braided into a spiral braid, wherein the number of filaments is twenty, with from one to ten filaments being of a first color and the remaining filaments being of a second color different from said first color.

5. The surgical suture of claim 2 braided into a lattice braid, wherein said suture in cross-section exhibits a generally rectangular shape, with filaments traversing said ends of said first and second paths being located at the corners of said rectangular shape.

6. The surgical suture of claim 2 braided into a lattice braid, wherein each moving filament dispenser alternately passes over then under the filaments dispensed on the paths it intersects.

7. The braided surgical suture of claim 2 braided into a lattice braid, wherein there are at least three filament dispensers traversing each of said paths.

8. The surgical suture of claim 2 braided into a lattice braid, wherein there are formed a plurality of core filament passageways located adjacent to the points of intersection of two or more of said paths.

9. The surgical suture of any one of claims 2 or 5 to 8 braided into a lattice braid, wherein the filament of one of said first and second paths is a first color and the filament of the other of said first and second paths and the filament of said third path is a second color different from said first color, or wherein the filament of said third path is a first color and the filaments of said first and second paths is a second color different from said first color.

10. The surgical suture of any one of claims 2 or 5 to 8 braided into a lattice braid, wherein the filament of said first path is a first color, the filament of said second path is a second color different from said first color and the filament of said third path is a third color different from said first and second colors.
